Europäisches Patentamt

European Patent Office

Office européen des brevets

(11) Publication number: **0 061 549**
**B1**

(12) **EUROPEAN PATENT SPECIFICATION**

(45) Date of publication of patent specification: **20.03.85**

(21) Application number: **81303601.9**

(22) Date of filing: **06.08.81**

(51) Int. Cl.⁴: **C 12 N 5/00**

(54) **Collagen tissue culture substrate.**

(30) Priority: **27.03.81 JP 46021/81**

(43) Date of publication of application:
**06.10.82 Bulletin 82/40**

(45) Publication of the grant of the patent:
**20.03.85 Bulletin 85/12**

(84) Designated Contracting States:
**CH DE FR GB IT LI NL SE**

(56) References cited:
**US-A-3 156 620**
**US-A-4 169 761**

**CHEMICAL ABSTRACTS, vol. 74, no. 21, May 24, 1971, page 82, abstract no. 108057r, Columbus, Ohio (US) M.A. KARASEK et al.: "Growth of postembryonic skin epithelial cells on collagen gels" & J. Invest. Dermatol. 1971, 56(3), 205-10**
**CHEMICAL ABSTRACTS, vol. 80, no. 19, May 13, 1974, pages 220-221 abstract no. 106115h, Columbus, Ohio (US) G.O. GEY et al.: "Long-term growth of chicken fibroblasts on a collagen substrate" & Exp. Cell. Res. 1974, 84(1-2), 63-71**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Kato, Tadaaki**
**K-407, 3-27 Nakadai**
**Itabashi-ku Tokyo (JP)**
Inventor: **Hirai, Tohru**
**5-31-1 Kasumigaseki-Kita**
**Kawagoe-shi Saitama-ken (JP)**
Inventor: **Ando, Takao**
**3-10-13 Nerima**
**Nerimaku Tokyo (JP)**
Inventor: **Yoshimura, Makoto**
**7-23-16 Ryoke**
**Urawa-shi Saitama-ken (JP)**
Inventor: **Yoshikumi, Chikao**
**2-19-46 Higashi**
**Kunitachi-shi Tokyo (JP)**
Inventor: **Matsunaga, Kenichi**
**3-26-1 Hyakunin-cho**
**Shinjuku-ku Tokyo (JP)**

(74) Representative: **Myerscough, Philip Boyd et al**
**J.A.Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

(56) References cited:
CHEMICAL ABSTRACTS, vol. 93, no. 23,
December 8, 1980, page 319, abstract no.
218271f, Columbus, Ohio (US) J.A. HUW et al.:
"The effect of different collagen types used as
substrata on myogenesis in tissue culture" &
Cell. Biol. Int. Rep. 1980, 4(9), 841-50
BIOLOGICAL ABSTRACTS, vol. 56, 1973,
abstract no. 17340 E.B. MASUROVSKY:
"Photo-reconstituted collagen gel for tussue
culture substrates" & Exp. Cell. Res. 76(2):
447-448 1973

## Description

This invention relates to a tissue culture substrate, particularly for monolayer culture, which comprises a soluble collagen, its use in tissue culture and the soluble collagen and its preparation.

Monolayer culture is tissue culture in which cells isolated from a living body, tissue pieces or microorganisms are cultured under artificial condition. The technique of monolayer culture utilises the adhesive property of cells onto the wall surfaces of a culture vessel. The method is suitable for the culture of cells requiring a solid support (substrate) for their growth, such as fibroblasts and epithelial cells which form sheets or fibers unless they deform or denature.

Since a planar colony of cells is formed in the monolayer culture, the state of cells can be observed by a microscope at any time. Accordingly, monolayer culture is advantageous for studies depending on the morphology of cells, for example, studies on the changes of cells or the induction of differentiation by viral infection, radioactive irradiation or anticancer drugs.

The material of a solid support, such as a vessel and a film, for monolayer culture should satisfy the following requirements:

(1) be a non-stimulant to cells,

(2) not swell, not dissolve in and not be corroded by culture liquid, and

(3) be easily sterilized and hardly deform or denature during sterilization.

From these viewpoints, metal, glass, earthenware or synthetic polymeric material has hitherto been utilized. However, the tissue culture substrates conventionally used are not excellent as far as cell contact is concerned, particularly for cells in primary culture. Accordingly, they are unsatisfactory substrates for monolayer culture.

Collagen derived from a living body has been proposed as a material for a tissue culture substrate (refer to R. L. Ehrmann et al., J. Nat. Cancer Inst., Vol. 16(6), page 1375, 1956). The collagen is preferably transparent for observation by an inverted microscope of the state of cells in culture. Acid-soluble collagen extracted with an acidic solvent from rat-tail tendon has been used. Native acid-soluble collagen, however, is limited in amount. The preparation of acid-soluble collagen is complicated. Accordingly, native acid-soluble collagen is not readily available at a low price and is used only in laboratories.

Rather than native acid-soluble collagen, the use of collagen solubilized by a treatment with an enzyme, which has been used for a blood coagulant, has been proposed. However, it is difficult to remove completely a protease from the solubilized collagen. Such collagen is not preferred as a tissue culture substrate. Moreover, the enzyme-treated collagen is expensive. The use of collagen is therefore restricted because of the lack of availability, unsatisfactory quality and cost of the collagen.

According to the present invention, there is provided a tissue culture substrate comprising a soluble collagen which has a denaturation temperature of 31 to 40°C, an S-constant of 1.12 to 1.62 and an intrinsic viscosity of 1.8 to 10.4 dl/g. The "soluble collagen" of the invention is collagen which may be soluble in an aqueous solution of an acid. The soluble collagen is readily available and preferably contains 312 to 340 glycine residues, 119 to 138 proline residues, 94 to 100 hydroxyproline residues and 2.6 to 5.5 tyrosine residues per 1000 amino residues thereof.

The soluble collagen for use in the invention is different from the above-mentioned native acid-soluble collagen and enzyme-treated collagen in its physical properties and/or amino acid composition. Native acid-soluble collagen has an S-constant of 1.65 and an intrinsic viscosity of 12.5 dl/g. Enzyme-treated collagen has an S-constant of 1.10 and an intrinsic viscosity of 1.6 dl/g, and contains fewer tyrosine residues (for example, 0.5 to 2 tyrosine residues per 1000 amino acid residues).

The denaturation temperature is a collagen-to-gelatin transition temperature. This temperature was determined by a differential scanning calorimeter (Model DSC-1B, manufactured by Perkin Elmer Company, USA).

The S-constant is a value depending on the state of base units of collagen and is an important factor relating to several physical properties. In particular, the transparency of the tissue culture substrate of collagen decreases with increase of the S-constant of the soluble collagen. Soluble collagen with an S-constant or more than 1.62 may not be inconvenient for a tissue culture substrate, but tissue culture substrates comprising such soluble collagen cannot be utilized in practice because of their low transparency. The soluble collagen of the invention has an S-constant between that of native acid-soluble collagen and that of enzyme-treated collagen. The S-constant is a sedimentation constant measured by using a tester. Model MOM 3170/b (made by MOM Company, Hungary), and a solution of collagen of 2 mg/ml at 20°C.

The intrinsic viscosity is a factor relating to the entanglement and molecular weight of collagen molecules, etc.. Intrinsic viscosity was determined at 24.8°C and by using 0.15 M citric acid buffer at pH of 3.7 as a solvent.

The amino acid composition of collagen was obtained by using the amino acid analyzer (Model JLC-6AH, manufactured by Nippon Electron Co., Japan).

In the invention, native connective tissues of animals such as cattle, pigs and whales, for instance their hides, tendons, intestines or bones, are used as a crude material of collagen. Preferably, raw hides, or refrigerated, salted or dried hides are used because of their high collagen content.

The soluble collagen of the invention may be preferably prepared by irradiating an aqueous acidic dispersion of insoluble collagen with ultrasonic waves. "Insoluble collagen" is collagen which is insoluble in an aqueous solution of an acid or a salt.

The pH of the aqueous acidic dispersion is preferably from 2 to 4. When the pH is lower than 2, it is not favourable since, once swollen, insoluble collagen fibers may deposit. When the pH is between 4 and 10, a uniform dispersion can not be obtained. Insoluble collagen fibers are not sufficiently swollen to sediment at the bottom of the vessel for dispersion when left still. When the pH is higher than 10, denaturation of insoluble collagen fibers occurs.

The collagen content of the aqueous acidic dispersion is preferably 0.5 to 100 mg/ml, more preferably 0.5 to 50 mg/ml. Where the collagen content is higher than 100 mg/ml, the viscosity of the dispersion is so high that handling of the dispersion is difficult. On the other hand, where the collagen content is lower than 0.5 mg/ml, although there are no problems concerning the operation and the quality of the product, it is not favorable because of the labour costs for separating and recovering the product are too high.

As the acid for preparing the aqueous acidic dispersion of insoluble collagen, an inorganic acid such as hydrochloric, phosphoric or sulfuric acid, or an organic acid such as acetic, butyric, citric, lactic, succinic or tartaric acid, or a mixture thereof may be used.

The amount of energy supplied to the aqueous acidic dispersion by the irradiation of ultrasonic waves is preferably 108 to 2500 Kcal per liter of dispersion. The frequency of the waves is not restricted provided of course that it is in the range for so-called ultrasonic waves. However, the frequency is preferably in the range of 17.5 to 24.5 KHz. The amount of energy irradiated is determined by the desired value of the S-constant. The crude collagen material in the dispersion is gradually fibrated or solubilized as irradiation progresses resulting eventually in the separation of the collagen into individual molecules. Such a change of state can be monitored by measuring the S-constant of the dispersion. The S-constant decreases gradually as the irradiation progresses, eventually until the lower limit of the S-constant of 1.12 is reached.

The temperature of the dispersion during irradiation is kept at a temperature at which no thermal denaturation of collagen is induced. The temperature should therefore be lower than 30°C, preferably lower than 20°C. On carrying out the irradiation, the dispersion, which was extremely dense, viscous and opaque at the beginning, becomes less viscous and more transparent with the progress of solubilization of the collagen by the irradiation.

The irradiation of ultrasonic waves onto the dispersion can solubilize almost all the crude insoluble collagen and is effected without any contamination by undesired substances. Accordingly, a new material for a tissue culture substrate can be supplied easily and abundantly. This material can take the place of native acid-soluble collagen which is restricted in quantity.

Besides, it is obvious from the values of the S-constant and intrinsic viscosity of the tissue culture substrate that fluctuations in specific properties of the native crude collagen material due to individual differences, such as differences between species, sexes and ages of the aminal from which the collagen was derived, have been homogenized by the irradiation treatment.

It may be particularly advantageous to subject the crude collagen material to the following pretreatment before preparing an aqueous acidic dispersion of insoluble collagen for use in the production of the soluble collagen.

By the pre-treatment, impurities such as proteins other than collagen, for instance albumin and globulins, and lipids, are removed from the crude collagen which is obtained by conventional treatment such as depilation and decalcification of the above-mentioned crude collagen material. The removal of proteins other than collagen is effected by repeatedly immersing the crude collagen in an aqueous saline solution. The lipids in the crude collagen can be removed by extracting the crude collagen with a mixed solvent, for instance acetone-ethanol or aqueous acetone-ethanol.

Further, neutral-salt soluble collagen, which is soluble in an aqueous solution of a salt, may be removed by extraction with an aqueous phosphoric buffer solution containing $Na_2HPO_4$—$KH_2PO_4$ and the like. Acid soluble collagen may be removed by extraction with a buffer solution containing citric acid-potassium hydrogen citrate ($KH_2C_6H_5O_7$) or a phosphoric buffer solution containing citric acid-$Na_2HPO_4$. The pH, the concentration of the buffer solutions and the combination of acid and salt in the buffer solutions can be suitably changed according to the components to be removed.

The conditions of the above-mentioned pre-treatment, i.e. extraction process, are not restricted. However, the extraction is ordinarily carried out at a temperature of less than 5°C for one or several days.

The thus pre-treated crude collagen (insoluble collagen) substantially shows no ultraviolet absorption in the range of 250 to 290 mm, and has a lipid content of less than 0.5% by weight.

The aqueous acidic dispersion of insoluble collagen which has been irradiated by ultrasonic waves can be treated by known methods such as neutralization, dialysis and freeze-drying to form a tissue culture substrate. The tissue culture substrate of the invention can be used in the form of films or gels. It can be applied without any hindrance to vessels, for instance Petri dishes, vials, bottles, flasks, test tubes, plates or beakers, for use in tissue culture. There are no restrictions on the material of the vessels. They may be of glass or plastics, for example.

Embodiments for preparing for a tissue culture substrate are described below:

### 1. Film

An aqueous solution of collagen at a concentration of 3 mg/ml was added dropwise to a Petri dish for tissue culture. After drying in air the dish was left in an ammoniacal atmosphere. Then the dish was washed well with sterilized water and dried to form a tissue culture substrate in the form of a film.

### 2 Gel

2-1) The same solution as in 1. was put into a Petri dish for tissue culture. The dish was left overnight in an ammoniacal atmosphere. Then, after letting the ammonia to evaporate off, the dish was washed well with sterilized water and stored under refrigeration.

2-2) One part by volume of an aqueous 2.0 M solution of sodium chloride containing 0.1 M of disodium hydrogen phosphate was mixed with 10 parts by volume of the same solution as in 1. After adjusting the pH of the mixture by adding an aqueous 0.1 M solution of sodium hydroxide to 7.4 to 7.6, the mixture was stored under refrigeration.

### 3. Freeze-dried substrate:

After freeze-drying the gel preparation obtained in 2, it was stored. On use, sterilized water or a culture medium was added to the stored gel to form a solution. The solution was again gelled under refrigeration.

In addition, on carrying out tissue culture, aseptic operation should be effected as far as possible. It is preferable to add an antibiotic such as penicillin or streptomycin to the culture medium. Furthermore, in the case where the tissue culture substrate is on the surface of a matrix, for instance granular plastics, glass or metal, such a substrate can be applied to suspension culture.

The tissue culture substrate of the invention is suitable for culturing the cells taken from a living body which grow as sheets or filaments. The cells can be derived from animals including mammals, birds, reptiles, amphibians, fishes and insects. Such cells are derived from epithelial tissue, tissue within the connective tissue and nervous tissue. The tissue culture substrate is particularly suitable for culturing dermal and muscular fibroblasts derived from mammals including humans. The substrate is suitable for the culture of cells derived from human cancers such as HeLa cells, KB cells, Detroit-6 cells, and for the primary culture of human cancer cells. The method of cell culture is not restricted and can be effected by known methods and means. The following Example illustrates the invention.

### Example:

Fifty grams (dry-weight of 12.5 g) of finely cut stear hide, native to North America, cut into approximate 5 mm squares consisting of crude collagen was immersed in 500 ml of an aqueous solution of hydrogen chloride at pH of 2.5 for a night to cause the collagen to become acid-swollen. The acid-swollen pieces of the crude collagen were dispersed in 2500 ml of an aqueous solution of hydrogen chloride at pH of 3 using a juice mixer. After adjusting the pH to 3 by adding concentrated hydrochloride acid, an aqueous dispersion containing 5 mg of collagen per milliliter was obtained. The aqueous acidic dispersion was subjected to ultrasonic wave treatment by supplying the aqueous acidic dispersion continuously to the top of a nozzle of an ultrasonic wave-generator (manufactured by Kaijo Denki Co., type of WA-6283, output of 600 W, frequency of 19.5 KHz) while keeping the temperature of the aqueous dispersion at 15°C by cooling the vessel. The amount of energy irradiated was 1000 Kcal per liter of the dispersion. By subjecting the thus obtained aqueous acidic solution to conventional steps of neutralization and dialysis, a tissue culture substrate according to the invention was obtained.

The attachment rates of cultured cells were compared as follows using three kinds of substrates; (1) film and gel substrates prepared by using the soluble collagen of the invention obtained above, (2) film and gel substrates prepared by using native acid-soluble collagen and (3) Petri-dishes (Falcon) not having any collagen and made of polystyrene, 35 mm in diameter.

10 Ml of MEM-culture medium containing 10% of fetal calf serum was placed in each Petri-dish. After 1 hour at 37°C, 1 ml of the MEM culture medium containing $1 \times 10^4$ of HeLa cells was further charged into each Petri-dish. The cells were cultured for 2 hours at 37°C under an atmosphere of carbon dioxide. Then, the attachment rate was determined by counting the number of cells floating within the culture liquid in the Petri-dish. The results are shown in Table 1. The major properties of the materials used for preparing the substrates in the Petri-dishes are shown in Table 2.

TABLE 1

| % of cells attached | | |
|---|---|---|
| Example | Collagen film | 84.5 |
| | Collagen gel | 88.3 |
| Comparative Example | native acid-soluble collagen, film | 87.0 |
| | native acid-soluble collagen, gel | 91.4 |
| Petri-dish (Falcon) without film or gel | | 38.6 |

TABLE 2

| Material | Amino acid composition | | | | Denaturation temperature (°C) | S-constant | Material | Intrinsic viscosity $[\eta]$ |
|---|---|---|---|---|---|---|---|---|
| | Gly | Pro | Hyp | Tyr | | | | |
| Present invention | 335 | 122 | 96 | 5.0 | 34 | 1.42 | Present invention | 8.2 |
| Native acid-soluble collagen | 320 | 126 | 85.6 | 4.9 | 37 | 1.65 | Native acid-soluble collagen | 12.5 |

As is seen in Table 1, the attachment rate of cells onto the substrates of the invention is far better than onto the Petri-dish of polystyrene and is similar to that of the substrates comprising the native acid-soluble collagen.

## Claims

1. A tissue culture substrate comprising soluble collagen, characterised in that the soluble collagen has a denaturation temperature of 31 to 40°C, a S-constant of 1.12 to 1.62 and an intrinsic viscosity of 1.8 to 10.4 dl/g.

2. A tissue culture substrate according to claim 1, in which the soluble collagen contains 2.6 to 5.5 tyrosine residues per 1000 amino acid residues thereof.

3. A tissue culture substrate according to claim 1 or 2, which is in the form of a film or gel.

4. A tissue culture substrate according to any one of the preceding claims, in which the soluble collagen has been prepared by irradiating an aqueous acidic dispersion of insoluble collagen with ultrasonic waves.

5. A tissue culture substrate according to claim 4, in which the pH of the aqueous acidic dispersion is from 2 to 4.

6. A tissue culture substrate according to claim 4 or 5, in which the collagen content of the aqueous acidic dispersion is 0.5 to 100 mg/ml.

7. Use in tissue culture of a substrate as claimed in any one of the preceding claims.

8. A soluble collagen suitable for use as a tissue culture substrate characterised in that the soluble collagen has a denaturation temperature of 31 to 40°C, a S-constant of 1.12 to 1.62 and an intrinsic viscosity of 1.8 to 10.4 dl/g.

9. A process for preparing a soluble collagen suitable for use as a tissue culture substrate, characterised in that an aqueous acidic dispersion of insoluble collagen is irradiated with ultrasonic waves to obtain a soluble collagen having a denaturation temperature of 31 to 40°C, a S-constant of 1.12 to 1.62 and an intrinsic viscosity of 1.8 to 10.4 dl/g.

## Patentansprüche

1. Gewebekultur-Substrat mit einem Gehalt an löslichem Kollagen, dadurch gekennzeichnet, daß das lösliche Kollagen eine Denaturierungstemperatur von 31 bis 40°C, eine S-Konstante von 1,12 bis 1,62 und eine Eigenviskosität von 1,8 bis 10,4 dl/g aufweist.

2. Gewebekultur-Substrat nach Anspruch 1, dadurch gekennzeichnet, daß das lösliche Kollagen 2,6 bis 5,5 Thyrosinreste je 1000 Aminosäureresten enthält.

3. Gewebekultur-Substrat nach Anspruch 1 oder 2, dadurch gekennzeichnet, daß es in Form einer Schicht oder eines Gels vorliegt.

4. Gewebekultur-Substrat nach einem der vorangehenden Ansprüche, dadurch gekennzeichnet, daß das lösliche Kollagen durch Beschallen einer wässrigen sauren Dispersion von unlöslichem Kollagen mit Ultraschallwellen hergestellt wurde.

5. Gewebekultur-Substrat nach Anspruch 4, dadurch gekennzeichnet, daß der pH-Wert der wässrigen sauren Dispersion 2 bis 4 beträgt.

6. Gewebekultur-Substrat nach Anspruch 4 oder 5, dadurch gekennzeichnet, daß der Kollagengehalt der wässrigen sauren Dispersion 0,5 bis 100 mg/ml beträgt.

7. Verwendung eines Substrats nach jeglichem der vorangehenden Ansprüche in einer Gewebekultur.

8. Lösliches Kollagen zur Verwendung als Gewebekultur-Substrat, dadurch gekennzeichnet, daß das lösliche Kollagen eine Denaturierungstemperatur von 31 bis 40°C, eine S-Konstante von 1,12 bis 1,62 und eine Eigenviskosität von 1,8 bis 10,4 dl/g aufweist.

9. Verfahren zur Herstellung von löslichem Kollagen zur Verwendung als Gewebekultur-Substrat, dadurch gekennzeichnet, daß man eine wässrige saure Dispersion von unlöslichem Kollagen mit Ultraschallwellen beschallt, um lösliches Kollagen mit einer Denaturierungstemperatur von 31 bis 40°C, einer S-Konstanten von 1,12 bis 1,62 und einer Eigenviskosität von 1,8 bis 10,4 dl/g zu erhalten.

## Revendications

1. Substrat pour la culture de tissus constitué de collagène soluble, caractérisé par le fait que le collagène soluble a une température de dénaturation de 31 à 40°C, une constante S de 1,12 à 1,62 et une viscosité intrinsèque de 1,8 à 10,4 dl/g.

2. Substrat pour la culture de tissus selon la revendication 1, caractérisé par le fait que le collagène soluble contient de 2,6 à 5,5 résidus de tyrosine pour 1000 résidus d'amino-acides.

3. Substrat pour le culture de tissus selon l'une quelconque des revendications 1 ou 2, caractérisé par le fait qu'il est sous forme d'un film ou d'un gel.

4. Substrat pour la culture de tissus selon l'une quelconque des revendications précédentes, caractérisé par le fait que le collagène soluble a été préparé par irradiation d'une dispersion aqueuse acide de collagène insoluble à l'aide d'ondes ultrasoniques.

5. Substrat pour la culture de tissus selon la revendication 4, caractérisé par le fait que le pH de la dispersion aqueuse acide est de 2 à 4.

6. Substrat pour la culture de tissus selon l'une quelconque des revendications 4 ou 5, caractérisé par le fait que la teneur en collagène de la dispersion aqueuse acide est de 0,5 à 100 mg/ml.

7. Utilisation pour la culture de tissus d'un substrat tel que revendiqué selon l'une quelconque des revendications précédentes.

8. Collagène soluble destiné à être utilisé comme substrat pour la culture de tissus, caractérisé par le fait que le collagène soluble a une température de dénaturation de 31 à 40°C, une constante S de 1,12 à 1,62 et une viscosité intrinsèque de 1,8 à 10,4 dl/g.

9. Procédé pour la préparation d'un collagène soluble destiné à être utilisé comme substrat pour la culture de tissus, caractérisé par le fait qu'une dispersion aqueuse acide de collagène insoluble est irradiée à l'aide d'ondes ultrasoniques pour obtenir un collagène soluble ayant une température de dénaturation de 31 à 40°C, une constante S de 1,12 à 1,62 et une viscosité intrinsèque de 1,8 à 10,4 dl/g.